# EUROPEAN PATENT APPLICATION

(11) **EP 1 629 830 A1**
(43) Date of publication of application: **01.03.2006**
(21) Application number: 03781153.6
(22) Date of filing: 30.05.2003
(51) Int. Cl.: A61K 8/98, A61K 8/97, A61K 8/44, A61Q 19/00

(54) **COSMETIC PREPARATION**

(71) Applicant: Kostelev, Nikolai Alexeevich, Dolgoprudny, Moskovskaya obl. 141700 (RU)
(72) Inventor: Kostelev, Nikolai Alexeevich, Dolgoprudny, Moskovskaya obl. 141700 (RU)
(74) Representative: Vannini, Torquato
(86) International application number: PCT/RU2003/000249
(87) International publication number: WO 2004/105720

(57) **Abrégé**

The invention relates to cosmetology. The inventive cosmetic preparation contains a physiologically acceptable base in the form of a disodium salt of ethylene diamine-tetracetic acid and a polyatomic low-molecular alcohol, and natural biologically active substances inthe form of an oily extract of sea-urchin roe and vegetable extracts at the following component ratio: 0.05-1.5 mass % oily extract of sea-urchin roe, 0.5-3.0 mass % vegetable extracts, 0.05-0.2 mass % disodium salt of ethylene diamine-tetracetic acid and 3.0-11.4 polyatomic low-molecular alcohol. In addition, said cosmetic preparation can comprise, according to the use thereof, urea, biohyaluronic acid, collagen hydrolysate, sodium ascorbyl phosphate, cellulose esters, lecithin, beeswax, aristoflex AVC, vitamins A and F, and the polyatomic low-molecular alcohol in the form of trietanolamine, cetyl alcohol and isopropyl myristate.

## Description

### Domaine de l'invention

L'invention se rapporte à la cosmétologie et concerne les produits cosmétiques contenant des substances biologiquement actives d'origine animale qui peuvent être utilisés pour le soin de la peau sous forme de crèmes et de masques cosmétiques.

### Etat de l'art

Actuellement, dans la production des produits de beauté, on utilise de plus en plus souvent des substances biologiquement actives d'origine végétale et animale. Cela stimule les échanges circulatoires dans la peau et améliore son état physiologique.

On connaît l'existence de produits de soin de la peau sous forme de crèmes (de jour et de nuit) et masques contenant des substances biologiquement actives d'origine naturelle, particulièrement, une culture de bifidobactéries. (voir EP 0043128 A, 06.01.1982).

On connaît l'existence de produits cosmétiques qui contiennent, en qualité de substances biologiquement actives, de la laitance des salmonidés d'où l'on extrait de l'ADN et d'autres agents effectuant une action régénératrice sur la peau. (voir FR 2511243 A, 1983).

On connaît l'existence d'un produit cosmétique - supercrème cosmétique - contenant le ferment collagénase et, en supplément, les substances biologiquement actives d'origine naturelle et chimique, y compris des gonades d'oursin. (RU 2139039 C1, 10.10.1999)

Pourtant tous ces produits, ainsi que les autres cosmétiques existants, exercent une action insuffisante sur les mécanismes des processus de régénération cutanée à cause d'une amenée trop lente et insuffisante d'éléments nutritifs aux céllules de la peau.

L'objectif de la présente invention est de créer un produit cosmétique qui transporte les éléments nutritifs vers les céllules de la peau d'une façon plus afficace, qui ait une grande stabilité agrégative et une apparence plus attrayante. La composition du produit de l'invention a pour objectif : a) d'une part, de normaliser la régénération de la peau lors de l'utilisation de ce dernier sous forme de crème ou de masque cosmétique contenant des agents stabilisants, biologiquement actifs, conservateurs et d'autres ; b) d'autre part, d'enrichir les céllules de la peau oxygénante par l'utilisation de la composition indiquée sous forme de gel (masque pour les paupières). En outre, le produit déposé élargit la gamme de produits de beauté existants.

### Fond de l'invention

Le produit cosmétique en question a une base autorisée, contient des substances biologiquement actives d'origine naturelle et assure les avantages indiqués ci-dessus. Le produit en question se distingue par le fait qu'il a pour base l'acide éthylènediaminotétraacétique sel disodique et l'alcool polyatomique de petit poids moléculaire et, comme substances biologiquement actives d'origine naturelle, il contient de l'extrait huileux d'oeufs d'oursin et des extraits végétaux avec la proportion des composants suivante (en % en masse) : extrait huileux d'oeufs d'oursin : 0,05 - 1,5 ; extraits végétaux : 0,5 - 3,0 ; acide éthylènediaminotétraacétique sel disodique : 0,05 - 0,2 et alcool polyatomique de petit poids moléculaire : 3,0 - 11,4.

Si nécessaire, le produit peut contenir également d'autres agents qui augmentent la stabilité agrégative du composé, améliorent l'aspect extérieur et autres qualités d'usage du produit. Parmi ces composants, on peut citer : l'eau, la composition aromatique, des parfums cosmétiques, des substances conservatrices, des éthers de cellulose, des poudres inorganiques, des polyacrylates, des graisses ordinaires, le propylène-glycol, PEG, l'urée, l'acide hyaluronique, le menthol, l'huile de paraffine, la vaseline, le collagène hydrolysat, la lécithine, la cire d'abeille, l'Aristoflex AVC, le collagène hydrolysat, les vitamines A, C, E et F.

Les produits fabriqués d'après l'invention peuvent contenir des extraits végétaux suivants (à titre non exhaustif) : concombre, camomille, bouleau, houblon, et plantain.

Comme alcool polyatomique de petit poids moléculaire, le produit cosmétique déposé peut contenir (à titre non exhaustif) du triéthanolamine, de l'alcool cétylique ou de l'isopropyl myristate.

Les avantages de l'invention par rapport à l'état de la technique antérieure sont dus aux justes proportions entre les substances biologiquement actives d'origine naturelle et la base, ainsi qu'à l'utilisation d'oeufs d'oursin sous forme d'extrait huileux obtenu dans les conditions d'extraction délicates (régime thermique modéré, basse intensité d'agitation, etc.).

L'un des éléments de la présente invention est un produit cosmétique sous forme de crème qui exerce sur la peau une action normalisante et régénératrice grâce à une meilleure oxygénation, à une meilleure amenée des éléments nutritifs vers les céllules cutanées et à une proportion des composants plus réussie. En ce sens, le produit de l'invention a la formule suivante (en % en masse) : extrait huileux d'oeufs d'oursin : 0,05 - 1,5 ; extraits végétaux 0,5 - 3,0 ; acide éthylènediaminotétraacétique sel disodique : 0,05 - 0,2 et alcool polyatomique de petit poids moléculaire : 3,0 - 11,4.

Si nécessaire, la composition peut être complétée par des agents exerçant une action sur sa stabilité agrégative, son aspect extérieur et autres qualités d'usage du produit, par exemple : lécithine, cire d'abeille, collagène hydrolysat, Aristoflex AVC, vitamines A et F ; comme extraits végétaux (à titre d'exemple) : ceux de camomille, de houblon et de plantain ; et comme alcool polyatomique de petit poids moléculaire (à titre d'exemple) : de l'éthanolamine et/ou de l'alcool cétylique et/ou de l'isopropyl myristate.

Un autre élément de la présente invention est une composition cosmétique sous forme de gel utilisé comme masque oxygénant et masque pour paupières et qui exerce sur la peau une action normalisante et régénératrice grâce, entre autres, à l'oxygénation du tissu cutané et, probablement, à une meilleure amenée des éléments nutritifs vers les céllules de la peau, comme résultat d'une proportion des composants plus réussie. La composition du gel produit de l'invention est la même que celle de la crème cosmétique sitée ci-dessus. Si nécessaire, la composition du produit fabriqué d'après l'invention peut être complétée (à titre non exhaustif) par : de l'urée, de l'acide hyaluronique, du collagène hydrolysat, du sodium ascorbyl phosphate et des éthers de cellulose.

Comme extraits végétaux, le gel produit de l'invention peut contenir, par exemple, de l'extrait (des extraits) de houblon, de plantain, de concombre, de camomille et de bouleau.

Comme alcool polyatomique de petit poids moléculaire, le gel en question peut contenir, par exemple, de l'alcool cétylique et/ou du triethanolamine et/ou de l'isopropyl myristate.

Ainsi, on dépose un produit cosmétique contenant comme base de l'acide éthylènediaminotétraacétique sel disodique et de l'alcool polyatomique de petit poids moléculaire et en qualité de substances biologiquement actives d'origine naturelle, de l'extrait huileux d'oeufs d'oursin et des extraits végétaux avec la proportion des composants suivante (en % en masse) :

| | |
|---|---|
| extrait huileux d'oeufs d'oursin | 0,05 - 1,5 |
| extraits végétaux | 0,5 - 3,0 |
| acide éthylènediaminotétraacétique sel disodique | 0,05 - 0,2 |
| alcool polyatomique de petit poids moléculaire | 3,0 - 11,4 |

La composition indiquée peut être complétée par des agents exerçant une action sur la stabilité agrégative, l'aspect extérieur et autres qualités d'usage du produit, par exemple, de la lécithine, de la cire d'abeille, du collagène hydrolysat, de l'Aristoflex AVC, des vitamines A et F. Comme extraits végétaux, elle peut contenir, par exemple, ceux de camomille, de houblon et de plantain, et comme alcool polyatomique de petit poids moléculaire, elle peut contenir, par exemple, du triéthanolamine, de l'alcool cétylique et de l'isopropyl myristate.

La composition déposée peut être fabriquée sous forme de crème cosmétique et de gel utilisé comme masque oxygénant et masque pour paupières.

L'invention sera illustrée ci-dessous par des exemples concrets de l'exécution dont l'unique objectif est d'illustrer la reproductibilité de l'invention et qui ne peuvent pas être utilisés pour la restriction des revendications. Une personne du métier pourrait sans beaucoup d'efforts réaliser d'autres variantes du produit fabriqué d'après l'invention en question qui ne sont pas décrites dans les exemples concrets mais qui tombent néanmoins dans le domaine de la protection de la formule de l'invention ci-dessous.

### Mode d'élaboration du produit déposé

En premier lieu, on obtient de l'extrait huileux d'oeufs d'oursin. A cette fin, on mélange les oeufs d'oursin frais avec un extragent (mélange d'huiles d'origine végétale et animale - par exemple : de pêche, d'amande, etc. - en proportion 1:10 respectivement) dans un agitateur à rotation lente pendant environ 45-60 minutes ou bien on les homogénéise pendant environ 15-20 minutes. Après l'extraction, le mélange passe par un séparateur centrifuge ou une centrifugeuse discontinue à 5-7 mille tours par minute. L'extrait ainsi obtenu est embouteillé dans des récipients opaques et conservé dans un réfrigérateur à 5-8° C environ.

### Fabrication de la crème et du masque cosmétique

Les produits en question sont fabriqués par un mélange mécanique des produits de départ de la composition déposée effectué dans un ordre déterminé avec une homogénéisation ultérieure de la dispersion préalablement obtenue. Ensuite, le produit homogénéisé est embouteillé et emballé.

Le produit cosmétique ainsi obtenu contenant de l'extrait huileux d'oeufs d'oursin, des extraits végétaux, de l'acide éthylènediaminotétraacétique sel disodique et de l'alcool polyatomique de petit poids moléculaire, est un ensemble de substances complexe et biologiquement actif, capable d'effectuer sur l'épiderme une action normalisante, régénératrice et oxygénante plus efficace que celle des produits existants. Cela est favorisé par la présence dans l'extrait huileux d'oursin d'un ensemble de ferments de protéase, d'hyaluronidase, d'un ensemble équilibré par la nature de tous les acides aminés, de précieux acides gras polyinsaturés, adaptogènes naturels - stimulateurs biogéniques de la régénération de la peau et d'un ensemble complet de vitamines et de microéléments qui conservent toute leur activité biologique lors de l'extraction délicate des oeufs d'oursin frais décrite plus hauts.

### Description détaillée de l'invention

Pour préparer le produit cosmétique sous forme de masque, on utilisent les composants suivants (voir tableau 1).

**Tableau 1 Caractéristiques des matières premières**

| N° | Matières premières, matériaux ou semi-produits | GOST (normes d'Etat), TU (normes techniques), OST (normes sectorielles) et autres normes applicables* | Paramètres réglementés |
|---|---|---|---|
| 1. | Huiles végétales | Normes de la société fournisseur | Aspect extérieur : liquide visqueux transparent ; densité, kg/m3 : 800-900 |
| 2. | Alcool cétylique | Normes de la société fournisseur | Substance blanche pulvérulente, bien soluble dans les alcools. |
| 3. | Émulsificateurs du type Cremophor A6, A25, Luvitol | Normes de la société BASF | Substances visqueuses ou céracées. |
| 4. | Dimeticon | Normes de la société fournisseur | Liquide transparent incolore. |
| 5. | Isopropyl myristate | Normes de la société fournisseur | Liquide huileux et visqueux, non soluble dans l'eau |
| 6. | Propylène-glycol | Normes de la société fournisseur | Liquide transparent et peu visqueux à l'odeur caractéristique d'alcool, soluble dans l'eau. |
| 7. | Acide éthylènediaminotétraacétique sel disodique (EDTA, Trilon B) | Normes de la société fournisseur | Substance blanche pulvérulente, bien soluble dans l'eau. |
| 8. | Eau potable | GOST 2874-82 | Aspect extérieur : liquide transparent incolore et inodore ; Substances en suspension, mg/l, max. : 10 ; Crudité générale, mg-eq/l, max. : 5 ; pH : 6,5-8 |
| 9. | Menthol | Normes de la société fournisseur | Substance cristalline blanche avec une odeur forte |
| 10. | Substance conservatrice (Germaben, propyl et méthylparabens) | Normes des sociétés fournisseurs | Substances pulvérulentes ou solutions dans le propylène-glycol. |
| 11. | Parfums cosmétiques | Normes des sociétés fournisseurs | Liquides avec une odeur caractéristique. |
| 12. | Poudres inorganiques (bentonite, oxyde de silicium, oxyde de zinc, kaolin) | Normes des sociétés fournisseurs | Poudres blanches conformes à la documentation technique |
| 13. | Extrait d'oeufs d'oursin | | Extrait huileux |
| 14. | Collagène hydrolysat (Desaron) | Normes des sociétés fournisseurs | Liquide visqueux incolore |

Le masque cosmétique est fabriqué de façon suivante : on charge successivement dans le dispositif agitateur la quantité prescrite de propylène-glycol et de Trilon. On les mélange jusqu'à obtenir une solution transparente homogène.

Ensuite, on ajoute la quantité prescrite des composants du groupe A (d'abord l'huile, ensuite les substituts d'huile et enfin tous les autres composants des groupes B et C dans l'ordre fixé par le tableau 2. Le mélange et l'émulsification des phases aqueuse et huileuse sont effectués jusqu'à l'obtention d'une masse homogène. Le mélange est effectué à la température de 78 ± 2 °C pendant environ 7-10 minutes ; l'homogénéisation ultérieure : pendant environ 10-15 minutes. Le produit obtenu est embouteillé dans des récipients (flacons, fioles) et transporté au dépôt.

Au cas où le masque n'est pas conforme aux règles techniques, il est retourné pour une nouvelle homogénéisation. La réception du produit après cette deuxième homogénéisation est effectuée de façon analogique.

**Tableau 2 Pharmacopée de base du masque cosmétique**

| N° | Ingrédients | Quantité en % en masse |
|---|---|---|
| | | Masque collagène |
| A | Cremophor A6 | 2,2 |
| | Cremophor A25 | 2,5 |
| | Alcool cétylique | 3,0 |
| | Huile végétale | 3,0 |
| | Luvitol EHO | 2,0 |
| | Isopropyl myristate | 3,0 |
| | Dimeticon | 0,5 |
| B | EDTA | 0,1 |
| | Propylène-glycol | 3,0 |
| | Aqua | 70,35 |
| C | Extrait d'oeufs d'oursin | 0,3 |
| | Poudre inorganique | 6,0 |
| | Collagène hydrolysat (Desaron) | 3,0 |
| | Menthol | 0,05 |
| | Substance conservatrice : Germaben II | 0,8 |
| | Parfum cosmétique | 0,2 |

Dans la production des produits de beauté sous forme de gel, on utilisent des éthers de cellulose (méthylcellulose, hydroxyéthylcellulose, etc.), des polyacrylates (carbomers, carbopols), de l'eau, des huiles, des surfactants et d'autres agents exerçant une action sur la stabilité agrégative, l'aspect extérieur et autres qualités d'usage du produit.

Les caractéristiques des matières premières initiales, matériaux et semi-produits des gels sont données dans le tableau 3

**Tableau 3**

| N° | Matières premières, matériaux ou semi-produits | GOST (normes d'Etat), TU (normes techniques), OST (normes sectorielles) et autres normes applicables* | Paramètres réglementés |
|---|---|---|---|
| 1. | Éthers de cellulose (l'un des suivants) : - Méthylcellulose - Hydroxyéthylcellulose | Normes de la société Clariant (Allemagne) ou TU 6-05-1857-87 (Russie) | Matériau blanc pulvérulent avec une teneur en constituant basique min. 97 % en masse, degré de polymérisation min. : 500 |
| 2. | Polyacrylates (l'un des suivants) - Carbopol | Normes de la société fournisseur | Poudre blanche gonflant dans l'eau. |
| 3. | Triéthanolamine | Normes de la société fournisseur | |
| 4. | Triglycéride caprylique (Rofetan) | Normes de la société fournisseur | Liquide transparent et peu visqueux, non soluble dans l'eau. |
| 5. | Propylène-glycol | Normes de la société fournisseur | Liquide transparent et peu visqueux à l'odeur caractéristique d'alcool, soluble dans l'eau. |
| 6. | PEG-7 glyceryl cocoate | Normes de la société fournisseur | Liquide transparent et peu visqueux, soluble dans l'eau. |
| 7. | Acide éthylènediaminotétraacétique sel disodique (EDTA, Trilon B) | Normes de la société fournisseur | Substance pulvérulente blanche, bien soluble dans l'eau. |
| 8. | Urée | Normes de la société fournisseur | Substance cristalline blanche, bien soluble dans l'eau. |
| 9. | Extraits végétaux naturels | Normes de la société fournisseur | Extraits à base d'alcool ou d'huile. |
| 10. | Extrait d'oeufs d'oursin | | Extrait à base d'huile. |
| 11. | Composition aromatique | Normes de la société fournisseur | Liquide huileux avec une odeur caractéristique |
| 12. | Eau potable | GOST 2874-82 | Aspect extérieur : liquide transparent incolore ; Substances en suspension, mg/l, max. : 10 Crudité générale, mg-eq/l, max.:5; PH : 6,5-8 |
| 13. | Tocophérol acétate (vitamine E) | Normes de la société fournisseur | Liquide visqueux transparent, non soluble dans l'eau. |
| 14. | Acide hyaluronique (solution à 2 %) | Normes de la société fournisseur | Liquide visqueux incolore, bien soluble dans l'eau. |
| 15. | Collagène hydrolysat (Desaron) | Normes de la société fournisseur | Liquide visqueux incolore. |
| 16. | Sodium ascorbyl phosphate (vitamine C) | Normes de la société fournisseur | Substance pulvérulente blanche, soluble dans l'eau. |
| 17. | Substances conservatrices (Germaben, propyl et méthyl-parabens) | Normes de la société fournisseur | Substances pulvérulentes ou solutions dans le propylène-glycol. |

Les gels sont fabriqués par voie de mélange mécanique et successif des 25 ingrédients de départ en fonction de la pharmacopée demandée (d'abord, on charge dans le dispositif agitateur les composants du groupe A, ensuite B et enfin C) avec une homogénéisation ultérieure du mélange obtenu. Le gel homogénéisé est embouteillé, emballé et envoyé au magasin de produits finis.

La pharmacopée des produits de beauté sous forme de gel est présentée ci-dessous (voir tableau 4).

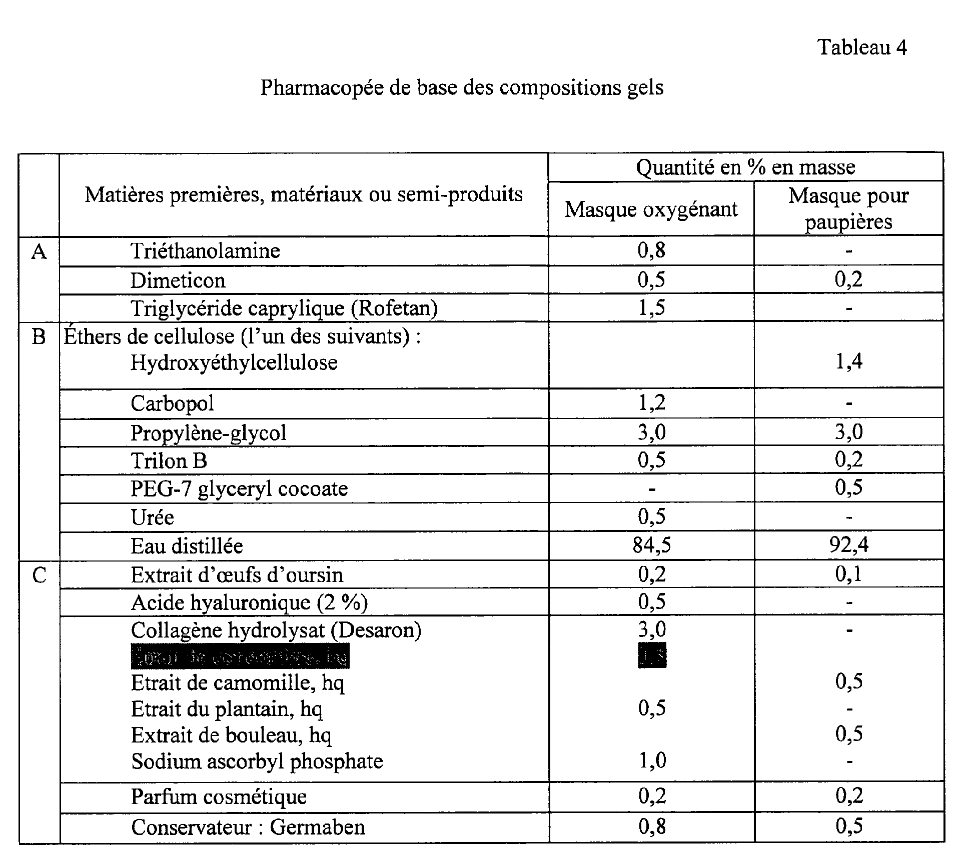

Dans la production des crèmes cosmétiques, on utilise de l'eau, des huiles, des surfactants, des polyélectrolytes solubles dans l'eau et d'autres agents exerçant une action sur la stabilité agrégative, l'aspect extérieur et autres qualités d'usage du produit.

Les caractéristiques des matières premières initiales, matériaux et semi-produits des crèmes cosmétiques sont donnés dans le tableau 5.

**Tableau 5 Caractéristiques des matières premières**

| N° | Matières premières, matériaux ou semi-produits | GOST (normes d'Etat), TU (normes techniques), OST (normes sectorielles) et autres normes applicables* | Paramètres réglementés |
|---|---|---|---|
| 1. | Huile de paraffine | Normes de la société fournisseur | Aspect extérieur : liquide visqueux transparent ; densité, kg/m3 : 800-880 |
| 2. | Huiles végétales | Normes de la société fournisseur | Aspect extérieur : liquide visqueux transparent ; densité, kg/m3 : 800-900 |
| 3. | Alcool cétylique | Normes de la société fournisseur | Substance pulvérulente blanche, bien soluble dans les alcools. |
| 4. | Vaseline | Normes de la société fournisseur | Masse blanche céracée, non soluble dans l'eau, fond à la température au-dessus de 45° C. |
| 5. | Citrate de sodium (disodiques et trisodiques) | Normes de la société fournisseur | Substance cristalline blanche, bien soluble dans l'eau. |
| 6. | Acide éthylènediaminotétraacétique sel disodique (EDTA, Trilon B) | Normes de la société fournisseur | Substance pulvérulente blanche, bien soluble dans l'eau. |
| 7. | Aristoflex AVC | Normes de la société fournisseur | Substance pulvérulente blanche, soluble dans l'eau. |
| 8. | Propylène-glycol | Normes de la société fournisseur | Liquide transparent et peu visqueux à l'odeur caractéristique d'alcool, soluble dans l'eau. |
| 9. | Émulsificateurs du type Cremophor A6, A25, Luvitol | Normes de la société BASF | Substances visqueuses ou céracées |
| 10. | Lécithine | Normes de la société fournisseur | Substance solide, insoluble dans l'eau. |
| 11. | PEG-7 Glyceryl cocoate | Normes de la société fournisseur | Liquide transparent et peu visqueux, soluble dans l'eau. |
| 12. | Isopropyl myristate | Normes de la société fournisseur | Liquide huileux et visqueux, non soluble dans l'eau |
| 13. | Eau potable | GOST 2874-82 | Aspect extérieur: liquide transparent incolore et inodore ; Substances en suspension, mg/l, max. : 10. Crudité générale, mg-eq/l, max. : 5 ; pH : 6,5-8 |
| 14. | Extraits végétaux naturels | Normes de la société fournisseur | Extraits à base d'alcool ou d'huile |
| 15. | Extrait huileux d'oeufs d'oursin | Normes de la société fournisseur | Extrait huileux |
| 16. | Tocophérol acétate (vitamine E) | Normes de la société fournisseur | Liquide visqueux transparent, non soluble dans l'eau. |
| 17. | Acide hyaluronique (solution à 2 %) | Normes de la société fournisseur | Liquide visqueux incolore, bien soluble dans l'eau. |
| 18. | Collagène hydrolysat (Desaron) | Normes de la société fournisseur | Liquide visqueux incolore. |
| 19. | Conservateur (Germaben, propyl et méthyl-parabens) | Normes des sociétés fournisseurs | Substances pulvérulentes ou solutions dans le propylène-glycol. |
| 20. | Parfums cosmétiques | Normes des sociétés fournisseurs | Liquides avec une odeur caractéristique. |

Le processus de fabrication de la crème cosmétique représente un mélange mécanique dans un ordre déterminé des substances initiales en fonction de la pharmacopée approuvée (d'abord on charge les composants A, ensuite B et enfin C) et une homogénéisation ultérieure de la dispersion obtenue. La crème homogénéisée est embouteillée, emballée et envoyée au magasin de produits finis.

La pharmacopée de base des crèmes est présentée dans le tableau 6.

**Tableau 6 Pharmacopée de base des crèmes**

| N° | Ingrédients | Quantité en % en masse | | |
|---|---|---|---|---|
| | | Crème normalisante | Crème régénérante | Crème oxygénante |
| A | Cremophor A6 | 2,0 | 2,0 | 2,0 |
| | Cremophor A25 | 2,0 | 2,0 | 2,0 |
| | Alcool cétylique | 3,0 | 3,0 | 3,0 |
| | Lanoline | 0,8 | 0,8 | 0,8 |
| | Vaseline | 0,5 | 0,5 | 0,5 |
| | Huile de paraffine | 1,0 | 1,0 | 1,0 |
| | Luvitol EHO | 3,0 | 3,0 | 3,0 |
| | Isopropyl myristate | 2,5 | 2,5 | 2,5 |
| | Dimeticon | 0,5 | 0,5 | 0,5 |
| | PEG-7 glyceryl cocoate | 1,2 | 1,2 | 1,2 |
| | Lécithine | 0,5 | 0,5 | 0,5 |
| | Cire d'abeille | 0,6 | 0,6 | 0,6 |
| B | EDTA | 0,05 | 0,05 | 0,05 |
| | Propylène-glycol | 3,0 | 3,0 | 3,0 |
| | Citrate de sodium | 0,5 | 0,5 | 0,5 |
| | Aristoflex AVC | 0,4 | 0,4 | 0,4 |
| | Aqua | 72,19 | 71,65 | 71,15 |
| C | D-Panthenol | 1,5 | 1,5 | 1,5 |
| | Extrait huileux d'oeufs d'oursin | 0,06 | 0,2 | 0,1 |
| | Extrait de camomille, hq | 1,5 | - | - |
| | Extrait huileux de houblon | - | 1,5 | - |
| | Extrait de plantain, hq | - | - | 1,5 |
| | Collagène hydrolysat (50 %) | 1,0 | 1,0 | 1,0 |
| | Tocophérol acétate | 0,8 | 0,8 | 2,0 |
| | Vitamine A | - | 0,4 | 0,5 |
| | Vitamine F | 0,4 | 0,4 | 0,4 |
| | Conservateur : Germaben II | 0,8 | 1,0 | 1,0 |
| | Parfum cosmétique | 0,2 | 0,2 | 0,2 |

### Tests biologiques

La gamme de produits de beauté à base d'extrait d'oursin est destinée à corriger les processus de vieillissement de la peau (produits «anti-âge»), à l'humidifier et à restituer son équilibre protéique et lipidique.

La gamme comprend les produits suivants :
- masque pour paupières avec extrait d'oursin ;
- crème pour la peau sèche et sensible avec extrait d'oursin ;
- crème régénérante avec extrait d'oursin ;
- crème oxygénante avec extrait d'oursin ;
- masque oxygénant avec extrait d' oursin ;
- masque de collagène avec extrait d'oursin.

La gamme est composée de produits professionnels à utiliser dans les salons de beauté sous forme de concentrés en ampoules : régénérant, vitaminant-régénérant; humidifiant-protecteur.

Les essais ont été effectués sur des sujets volontaires dans le salon *Polycorps cosmétique.*

### Exemple 1

Pour tester la qualité du produit contenant les composants indiqués au tableau 2, on a réalisé les tests sur des sujets volontaires âgés de 25 à 55 ans. Ces derniers ont subi un traitement au produit en question qu'on appliquait sur la peau du visage et du cou 2 à 3 fois par semaine pendant 15 à 20 minutes. Ensuite, on enlevait le produit au tampon et on rinçaient la peau traitée avec de l'eau d'abord tiède, ensuite froide. Une seule cure comprenait de 12 à 15 masques.

Le produit cosmétique en question améliorait l'état de tous les types de peau déjà après la première semaine d'utilisation en la rendant plus fraîche, lisse et élastique.

### Exemple 2

Pour tester la qualité du produit contenant les composants indiqués au tableau 4, on a réalisé les tests sur des sujets volontaires âgés de 25 à 55 ans. Ces derniers ont subi un traitement à deux produits : masque oxygénant et masque pour paupières. On appliquait le masque oxygénant sur tous les types de peau du visage et du cou pendant 20 minutes 1 fois par semaine. Ensuite, on enlevait le masque à l'eau tiède et on passait sur la peau traitée un cube de glace en décoction de menthe. Une cure comprenait de 10 à 12 masques. Le masque pour paupières était appliqué de la même façon.

L'effet était sensible déjà après la deuxième séance d'application.

Les tests des masques cosmétiques à base polymérique n'ont révélé aucune action toxique générale, irritante, sensibilisante ou allergisante. Les masques n'ont pas d'effets secondaires sur la peau, au contraire, ils exercent une action favorable sur son état.

### Exemple 3

Pour tester la qualité du produit contenant les composants indiqués au tableau 6, on a réalisé les tests sur des sujets volontaires âgés de 25 à 55 ans. Ces derniers ont subi un traitement aux crèmes nutritives et humidifiantes ayant une action normalisante, régénérante et oxygénante. Les crèmes de nuit nutritives avec une action normalisante, régénérante et oxygénante étaient appliquées sur la peau du visage que l'on laissait sur une peau grasse pendant 20 à 30 minutes, et sur une peau sèche pendant 1-1,5 heures. Ensuite, on les enlevait avec un tampon imbibé de lotion acidulée.

En comparaison avec les crèmes traditionnelles, on obtenait un effet déjà après 3 à 4 applications des crèmes en question. Les substances biologiquement actives pénétraient dans les couches dermiques plus profondes en rendant la peau plus élastique et en augmentant son tonus musculaire.

Les crèmes de jour humidifiantes avec une action normalisante, régénérante et oxygénante étaient appliquées sur le visage en une couche fine, les surplus étaient enlevés avec un tampon de coton. Depuis la première séance d'application, elles protégeaient activement la peau du visage des rayons solaires.

Ainsi, les tests ont démontré que les crèmes ci-dessus augmentent l'élasticité de la peau et la teneur en eau dans les céllules, stimulent tous les échanges circulatoires, ralentissent ceux de vieillissement cutané, etc.

Dans tous les cas d'utilisation de toute la gamme de produits contenant de 0,05 à 1,5 % en masse d'extrait huileux d'oeufs d'oursin, de 0,5 à 3,0 % en masse d'extraits végétaux, de 0,05 à 0,2 % en masse d'acide éthylènediaminotétraacétique sel disodique et de 3,0 à 11,4 % en masse d'alcool polyatomique de petit poids moléculaire, on a obtenu un effet positif beaucoup plus vite qu'avec les produits existants.

Le produit en question ne provoque aucun effet secondaire ni réactions allergiques.

Ainsi, le produit de l'invention peut être largement utilisé dans la cosmétologie comme un produit cosmétique destiné à normaliser et à corriger les processus de vieillissement de la peau, à l'humidifier et à restituer son équilibre protéique et lipidique.

## Revendications

1. Le produit cosmétique composé d'une base physiologiquement acceptable et de substances biologiquement actives d'origine naturelle, se distingue par le fait qu'il contient comme base de l'acide éthylènediaminotétraacétique sel disodique et de l'alcool polyatomique de petit poids moléculaire et qu'en qualité de substances biologiquement actives d'origine naturelle, il contient de l'extrait huileux d'oeufs d'oursin et des extraits végétaux avec la proportion des composants suivante (en % en masse) :
| | |
|---|---|
| Extrait huileux d'oeufs d'oursin | 0,05 - 1,5 |
| Extraits végétaux | 0,5 - 3,0 |
| Acide éthylènediaminotétraacétique sel disodique | 0,05 - 0,2 |
| Alcool polyatomique de petit poids moléculaire | 3,0 - 11,4 |

2. Le produit cosmétique indiqué au paragraphe 1 se distingue par le fait que le masque oxygénant contient en supplément de l'urée, de l'acide hyaluronique, du collagène hydrolysat, du sodium ascorbyl phosphate et, comme extraits végétaux, ceux de houblon et de plantain.

3. Le produit cosmétique indiqué au paragraphe 1 se distingue par le fait que le masque cosmétique pour paupières contient en supplément des éthers de cellulose et, comme extraits végétaux, ceux de concombre, de camomille et de bouleau.

4. Le produit cosmétique indiqué au paragraphe 1 se distingue par le fait que la crème cosmétique normalisante contient en supplément de la lécithine, de la cire d'abeille, de l'Aristoflex AVC, du collagène hydrolysat, des vitamines A et F et, comme extraits végétaux, celui de camomille.

5. Le produit cosmétique indiqué au paragraphe 1 se distingue par le fait que la crème cosmétique régénérante contient en supplément de la lécithine, de la cire d'abeille, du collagène hydrolysat, de l'Aristoflex AVC, des vitamines A et F et, comme extraits végétaux, celui de houblon.

6. Le produit cosmétique indiqué au paragraphe 1 se distingue par le fait que la crème cosmétique oxygénante contient en supplément de la lécithine, de la cire d'abeille, du collagène hydrolysat, de l'Aristoflex AVC, des vitamines A et F et, comme extraits végétaux, celui de plantain.

7. Le produit cosmétique indiqué au paragraphe 1 se distingue par le fait que, comme alcool polyatomique de petit poids moléculaire, il contient de préférence du triéthanolamine, de l'alcool cétylique et de l'isopropyl myristate.
